Europäisches Patentamt

European Patent Office

Office européen des brevets

(19)

(11) Numéro de publication: **0 234 149**
**B1**

(12) # FASCICULE DE BREVET EUROPEEN

(45) Date de publication du fascicule du brevet:
01.03.89

(51) Int. Cl.⁴: **C10M 159/20**

(21) Numéro de dépôt: **86402762.8**

(22) Date de dépôt: **11.12.86**

(54) Savons de calcium possédant une réserve de basicite élévée.

(30) Priorité: **30.12.85 FR 8519393**

(43) Date de publication de la demande:
**02.09.87 Bulletin 87/36**

(45) Mention de la délivrance du brevet:
**01.03.89 Bulletin 89/9**

(84) Etats contractants désignés:
**AT BE CH DE ES FR GB GR IT LI LU NL SE**

(56) Documents cités:
**FR-A- 1 540 384**
**FR-A- 2 424 927**

(73) Titulaire: **NORSOLOR S.A., Tour Aurore Place des Reflets, F-92080 Paris la Defense Cédex 5(FR)**

(72) Inventeur: **Marotel, Yves, 41 rue d'Herivaux, F-60580 Coye La Foret(FR)**
Inventeur: **Hurier, Francis, 8 Avenue du Clos vert Fleurines, F-60700 Pont Sainte Maxence(FR)**
Inventeur: **Mansot, Jean-Louis, La grande Penet Arnas, F-69400 Villefranche sur Saone(FR)**
Inventeur: **Martin, Jean-Michel, 21 rue du Clos Chapuis, F-69380 Lozanne(FR)**

(74) Mandataire: **Rieux, Michel, NORSOLOR Service Propriété Industrielle Tour Aurore Place des Reflets Cédex no. 5, F-92080 Paris la Défense 2(FR)**

ACTORUM AG

**Description**

La présente invention concerne de nouveaux savons de calcium possédant une réserve de basicité élevée plus communément appelés savons surbasés de calcium. Ces savons surbasés de calcium peuvent être utilisés dans de nombreuses applications, en particulier comme additifs anti-corrosion dans les lubrifiants. La présente invention a également pour objet une méthode de préparation de ces savons surbasés de calcium, ainsi que les compositions lubrifiantes contenant de tels savons surbasés de calcium.

Les savons surbasés de calcium les plus connus sont des sels d'acides alkylarylsulfoniques. Ce sont des composés difficiles à préparer et à mettre en œuvre. Un procédé classique de préparation de ces composés consiste à faire réagir un acide alkylarylsulfonique sur un oxyde ou un hydroxyde métallique dans une huile minérale. La réaction a lieu en présence de dioxyde de carbone (ou $CO_2$), et en présence de promoteurs facilitant la fixation du $CO_2$. Les promoteurs sont des composés à hydrogène mobile comme les phénols, les alcools, les aminoalcools. A l'issue de la réaction, on obtient une solution trouble que l'on purifie par centrifugation ou filtration. Le précipité ainsi obtenu contient notamment de l'acide sous forme de sel que l'on extrait afin de minimiser les pertes en acide (US-A 4 225 509). De tels procédés conduisent à des sels surbasés de calcium d'acides alkylarylsulfoniques possédant un TBN (ou Total Basic Number) supérieur ou égal à 200, et pouvant atteindre 350. Par TBN, on entend la réserve de basicité d'un savon surbasé. Le TBN d'un savon surbasé est l'équivalent en potasse correspondant à un gramme de savon lorsqu'on titre sa basicité par un acide fort. Il est exprimé en milligramme de potasse par gramme de sel surbasé de calcium. Cette valeur est définie conformément à la norme ASTM D 2896-73. Un autre inconvénient majeur des savons surbasés de calcium d'acides alkylarylsulfoniques réside dans le fait qu'ils donnent des solutions troubles rendant leur emploi malaisé.

La demanderesse a mis au point de nouveaux savons surbasés de calcium ne présentant pas les défauts des savons surbasés connus, qui possèdent un TBN élevé de l'ordre de 400, voire supérieur, et qui donnent lors de leur utilisation des solutions parfaitement stables et limpides.

Plus précisément, les savons surbasés de calcium selon l'invention sont caractérisés en ce qu'ils comprennent du calcium dissous dans au moins une huile sous la forme de carbonate et de sels d'acides, lesdits acides étant constitués d'un mélange ou non d'acides organiques carboxyliques saturés de 7 à 13 atomes de carbone possédant les caractéristiques suivantes:
– une teneur en acides linéaires comprise entre 0 et 40% en poids,
– une teneur en acides ramifiés sur le carbone 2 comprise entre 0 et 20% en poids,
– une teneur en acides mono ou polysubstitués sur le carbone 3 et/ou les carbones de rang supérieur, comprise entre 40 et 100% en poids.

Par huile au sens de la présente invention, on entend les huiles naturelles, les huiles semi-synthétiques, les huiles de synthèse. On peut citer, par exemple, les huiles d'origine animale ou végétale, les huiles minérales, les huiles liquides de pétrole telles que le kérosène, le gas oil, les essences de pétrole, les huiles lubrifiantes minérales, les huiles lubrifiantes synthétiques telles que les esters de diacides ou les esters de polyols. On peut également utiliser un mélange d'huiles naturelles et/ou d'huiles semi synthétiques et/ou d'huiles de synthèse choisies par exemple parmi celles citées ci-dessus. De préférence, on utilise les huiles liquides dans lesquelles les savons surbasés de calcium sont utilisés comme additifs. Les savons surbasés de calcium selon l'invention sont des concentrés de sels de calcium que l'on mélange facilement dans des compositions lubrifiantes. Les savons surbasés de calcium selon l'invention contiennent 5 à 80% en poids d'huile. De préférence les savons de calcium selon l'invention comprennent 30 à 60% en poids d'huile, afin de bénéficier à la fois d'un savon de calcium à réserve en alcalinité élevée et d'un produit parfaitement véhiculable.

De préférence, les savons surbasés de calcium sont préparés à partir d'acides organiques carboxyliques saturés en C8, C9, C10 (c'est-à-dire des acides comprenant respectivement 8, 9, ou 10 atomes de carbone) qui sont constitués de mélange d'isomères, et qui sont couramment appelés acides oxo. Ces acides oxo sont caractérisés par une faible teneur en acides linéaires, généralement inférieure ou égale à 10% en poids, une faible teneur en acides ramifiés sur le carbone 2, généralement inférieure ou égale à 10% en poids et une forte teneur en acides mono ou polysubstitués sur le carbone 3 et/ou les carbones de rang supérieur, généralement supérieure à 80% en poids. Les acides oxo sont obtenus par hydroformylation d'oléfines en C7, C8, C9 (c'est-à-dire des oléfines comprenant respectivement 7, 8, ou 9 atomes de carbone) suivie d'une oxydation.

De préférence encore, les savons de calcium selon l'invention sont préparés à partir de l'acide carboxylique saturé en C8 (c'est-à-dire comprenant 8 atomes de carbone) et commercialisé sous la marque CEKANOIC, cet acide étant constitué d'un mélange d'isomères de l'acides octanoïques contenant au plus 10% en poids d'acide n-octanoïque, au plus 10% en poids d'acide en C8 ramifiés sur le carbone 2 et d'au moins 80% en poids d'acides en C8 ramifiés sur le carbone 3 et/ou les carbones de rang supérieur. En effet, on a observé de manière surprenante que l'utilisation de cet acide permet d'obtenir des savons de calcium à basicité très élevée de l'ordre de 500 ou supérieure.

Parmi les acides organiques carboxyliques convenant également à la présente invention, on peut ajouter les dérivés mono ou polysubstitués en position 3 et/ou de rang supérieur des acides correspondants à l'acide heptanoïque, l'acide octanoïque, l'acide nonanoïque, l'acide décanoïque, l'acide undécanoïque, l'acide dodécanoïque. Il s'agit par exemple de l'acide 3-méthylhexanoïque, l'acide isooctanoïque, l'acide 4,5-diméthylhexanoïque, l'acide isononanoïque, l'acide 3,5,5-triméthylhexanoïque, l'acide isodécanoïque, l'acide 3-éthyloctanoïque, l'acide isoundécanoïque, l'acide 4-éthylnonanoïque, l'acide isododécanoïque. Convient également à la présente invention le mélange d'un ou de plusieurs des acides cités ci-dessus, en mélange ou non avec leurs isomères, étant entendu que la teneur en acides linéaires n'excède par 40% et que la teneur en acides substitués sur le carbone 2 n'excède pas 20%. La demanderesse a en effet trouvé que les acides linéaires et que les acides ramifiés sur le carbone 2 conduisent à l'obtention d'un produit visqueux, ou d'une prise en masse ou encore d'un précipité rendant le produit pratiquement inutilisable.

La présente invention a également pour objet un procédé de préparation des savons surbasés de calcium décrits ci-dessus, selon lequel on fait réagir sous agitation, un oxyde et/ou un hydroxyde de calcium avec du dioxyde de carbone (ou $CO_2$) que l'on fait barboter dans le milieu réactionnel et au moins un acide organique carboxylique, en présence d'au moins un promoteur favorisant la fixation du $CO_2$ et d'au moins un catalyseur, et en ce que l'on élimine l'eau formée au cours de la réaction. Le procédé selon l'invention se caractérise en ce que la réaction est effectuée dans au moins un solvant organique à une température comprise entre 80 et 120°C, et en ce que ledit acide est un acide organique carboxylique saturé, de 7 à 13 atomes de carbone, dont la teneur en acides linéaires est inférieure ou égale à 40% en poids, dont la teneur en acides ramifiés sur le carbone 2 est inférieure ou égale à 20% en poids, et dont la teneur en acides ramifiés sur le carbone 3 et/ou les carbones de rang supérieur est égale ou supérieure à 40% en poids.

En fin de réaction, après filtration, on substitue au solvant organique une huile, ou un mélange d'huiles, choisie parmi les huiles naturelles et/ou les huiles semi-synthétiques et/ou les huiles de synthèse. De préférence toutefois, l'huile choisie est celle qui est à la base de la composition lubrifiante dans laquelle le savon surbasé de calcium est additionné.

Conformément à un mode de réalisation préférée du procédé selon l'invention, pendant la réaction on permet l'évaporation du solvant organique que l'on recycle dans le milieu réactionnel de manière à y occasionner un barbotage favorable à la réaction.

Parmi les solvants organiques utilisables dans le procédé selon l'invention, on utilise au moins un solvant organique non polaire choisi parmi le naphta, l'hexane, le kérosène, le benzène, le toluène ou le xylène. On peut également utiliser un mélange d'hydrocarbures paraffiniques d'origine minérale ou de synthèse, comportant de préférence une faible proportion d'hydrocarbures aromatiques et/ou naphténiques tel que le white spirit. On peut également songer à utiliser des solvants organiques polaires comme les alcools, par exemple le butanol-1, le butanol-2, l'éthylène-glycol, le propylène-glycol, – l'éther monométhylique de l'éthylène glycol, l'éther diméthylique de l'éthylène glycol, le diéthylène glycol et ses éthers, des mélanges d'alcools dérivés de la paraffine ou la méthyléthylcétone.

Le rapport molaire calcium sur acide organique carboxylique utilisé lors de la réaction est généralement compris entre 0,55 et 2, ce qui correspond à une basicité comprise entre 1,1 et 4.

Rappelons que la basicité est égale au rapport d'équivalents calcium sur les équivalents d'acides carboxyliques utilisés, c'est-à-dire, au rapport molaire de la concentration de calcium sur celle d'acides carboxyliques multiplié par 2.

D'autre part, on impose un débit horaire de dioxyde de carbone tel que le rapport massique horaire de dioxyde de carbone sur le calcium soit compris entre 0,5 et 2, et de préférence entre 0,7 et 1,5.

Parmi les catalyseurs utilisables dans le procédé selon l'invention, on peut citer les oxydes métalliques, par exemple l'oxyde de zinc, l'oxyde d'aluminium $Al_2O_3$, l'oxyde d'argent $Ag_2O$, l'oxyde de magnésium MgO, les carboxylates de zinc comme l'octanoate de zinc.

Parmi les promoteurs facilitant la fixation du $CO_2$ et utilisables dans la présente invention, on peut citer les composés à hydrogène mobile comme les alcools, par exemple, le méthanol, le 2-propanol, l'alcool octylique, l'éthylène-glycol, le triéthylène-glycol, l'alcool stéarylique, l'alcool cyclohélène-glycol, l'alcool cyclohexylique, les alcools aromatiques comme le phénol; les amines, par exemple l'aniline, la phénylène-diamine, la dodécylamine; ou encore un mélange d'alcools et/ou d'amines, par exemple du méthanol et de l'ammoniaque.

De préférence toutefois, on utilise le méthanol avec lequel on obtient les basicités les plus élevées et les temps de filtration les plus courts lors de la préparation du savon de calcium selon l'invention.

Dans les savons surbasés de calcium selon l'invention, les promoteurs sont utilisés à raison de 1 à 25% en poids de sel de calcium final, et de préférence, à raison de 5 à 15%.

Les savons surbasés de calcium selon l'invention, en solution dans une huile, donnent des solutions stables et parfaitement limpides. De plus, ils possèdent une réserve de basicité (ou TBN) élevé, de l'ordre de 400, voire supérieur. Ils trouvent de nombreux emplois notamment en tant qu'additifs de lubrification. Ils peuvent également être utilisés comme catalyseurs d'oxydation ou intermédiaires dans la synthèse d'autres additifs de lubrification.

La présente invention sera mieux comprise à la lecture des exemples non restrictifs de l'invention qui vont suivre.

Dans les exemples suivants, pour la préparation du savon de calcium on utilise une huile paraffinique de dénomination commerciale 100 Neutral et une huile paraffinique de dénomination commerciale 200 Neutral.

Les caractéristiques physiques de ces 2 huiles sont données dans le tableau ci-dessous.

| Huile | Densité à 15°C | Point d'inflam- mabilité (°C) | Viscosité Engler à 50°C | Viscosité ab- solue en cSt à 37,8°C | Point de con- gélation (°C) | Indice d'acide (mg/KOG/g) |
|---|---|---|---|---|---|---|
| 100 Neut. | 865 | 200/220 | 2,3 | 20 | −12/−15 | 0,01 |
| 200 Neut. | 870/890 | 210 | 3,3/3,8 | 42 | −12/−15 | 0,05/01 |

## EXEMPLE 1

La préparation du savon surbasé de calcium est effectuée dans un réacteur équipé d'un système d'agitation mécanique de type ancre, d'un système de chauffage électrique, d'un appareil de distillation de type Dean Stark qui est relié à un condenseur avec séparation d'eau. Ce réacteur est branché sur un dispositif de recyclage principalement du solvant organique, issu du condenseur par l'intermédiaire d'une pompe pneumatique, dans le but de réaliser dans le milieu réactionnel un barbotage favorable à l'homogénéisation. Le réacteur est également équipé d'une arrivée de gaz carbonique. On introduit sous agitation mécanique dans le réacteur et à la pression atmosphérique la charge suivante (en parties en poids):
− white spirit: 286
− octanoate de zinc (à 10% en poids): 1,2
− triéthylène glycol: 35

Le mélange est porté à 70°C. On introduit ensuite dans le réacteur 128 parties d'hydroxyde de calcium à 97% en poids de pureté, puis après homogénéisation du mélange, on coule progressivement dans le réacteur 236 parties d'acide iso-octanoïque qui est constitué d'un mélange d'isomères comprenant moins de 10% en poids de ramifié sur le carbone 2 et comprenant 10% en poids de linéaire au maximum.

La réaction est exothermique et la température du mélange atteint 85°C. Ensuite, la température du mélange est portée à 95°C et on fait passer un courant de dioxyde de carbone avec un débit tel que le rapport massique par heure du dioxyde de carbone sur l'hydroxyde de calcium soit égal à 1,1 pendant 30 minutes. Après l'addition de dioxyde de carbone, on arrête la pompe aspirante qui a été maintenue en fonctionnement pendant la réaction. L'ensemble du montage est mis sous une pression de $1,33 \cdot 10^4$ Pa et la température dans le réacteur est portée à 120°C ce qui permet d'éliminer le reste d'eau présent dans le milieu réactionnel. Globalement, pendant toute la réaction on élimine 29,5 parties d'eau. On rétablit ensuite la pression atmosphérique dans le réacteur et on y ajoute 5 parties d'un adjuvant de filtration qui est dans le cas présent de la terre diatomée. Le mélange réactionnel est ensuite filtré à chaud (80°C) sous une pression de $4 \cdot 10^4$ à $7 \cdot 10^4$ Pa. On obtient 657 parties de sel de calcium qui se présente sous la forme d'une solution limpide jaune clair dont les caractéristiques sont les suivantes:
−  teneur en calcium: 10,3

− basicité (qui est égale à $\dfrac{2[Ca]}{[acide]}$): 2,04

− viscosité: 0,088 Pascal. seconde

Dans une seconde étape, on introduit dans un système d'évaporation 100 parties de ce sel surbasé de calcium et 14,2 parties d'une huile paraffinique de dénomination commerciale 200 Neutral représentative d'une base lubrifiante traditionnelle. On évapore le white spirit sous $1,33 \cdot 10^3$ Pa et à 110°C. On recueille 43,6 parties de white spirit.

Finalement on obtient 70,6 parties de savon surbasé de calcium qui se présente sous la forme d'un solide transparent de couleur ambrée et dont les caractéristiques sont les suivantes:
− masse volumique: 1,04 g/cm$^3$
− viscosité dynamique à 100°C: 0,5 Pascal. seconde
− Total Basic Number (TBN) (défini selon la norme ASTM D 2896-73): 400
− teneur en calcium (en % en poids): 14,4

## EXEMPLE 2

En suivant le mode opératoire décrit dans l'exemple 1, on fait réagir la charge suivante (en parties en poids):
− acide 3,5,5-triméthylhexanoïque: 259
− hydroxyde de calcium: 128
avec un débit de dioxyde de carbone tel que le rapport massique horaire de dioxyde de carbone sur l'hydroxyde de calcium est égal à 1,1 et en présence de (en parties en poids):
− triéthylène glycol: 35
− octanoate de zinc: 1,2
− white spirit: 263,3

Au cours de la réaction, on récupère 29,5 parties d'eau. En fin de réaction on recueille 671 parties de sel surbasé de calcium qui se présente sous la forme d'un liquide jaune clair limpide. Dans la seconde étape qui consiste à substituer la white spirit contenu dans le sel surbasé de calcium par une huile, qui est dans le cas présent un huile paraffinique de marque commerciale 200 Neutral, on ajoute dans 100 parties de sel surbasé 8,85 parties de cette huile. Après distillation, on recueille à 120°C et sous $1,33 \cdot 10^4$ Pa, d'une part 40 parties de white spirit, et d'autre part, 68,85 parties de sel surbasé de calcium sous la forme d'un solide couleur ambrée. Ses caractéristiques sont les suivantes:
– masse volumique: 1,046 g/cm³
– viscosité dynamique à 100°C: 4,25 Pascal.seconde
– total Basic Number (TBN): 381
– teneur en calcium (en % en poids): 13,4

EXEMPLE 3

En suivant le mode opératoire décrit dans l'exemple 1, on fait réagir la charge suivante (en parties en poids):
– acide commercialisé sous la marque CEKANOIC CK10 constitué d'un mélange d'isomères ramifiés d'acide décanoïque, sans ramification sur le carbone 2: 282
– hydroxyde de calcium: 128
avec un débit de dioxyde de carbone tel que le rapport massique horaire de dioxyde de carbone sur l'hydroxyde de calcium est égal à 1,1, et en présence de (en parties en poids):
– triéthylène glycol: 35
– white spirit: 254
– octanoate de zinc: 1,2
Au cours de la réaction , on récupère 29,5 parties d'eau. En fin de réaction, on recueille 670,7 parties de sel surbasé de calcium qui se présente sous la forme d'un liquide jaune clair limpide.
Dans la seconde étape qui consiste à substituer le white spirit contenu dans le sel surbasé de calcium par une huile qui est dans le cas présent une huile paraffinique de marque commerciale 200 Neutral, on ajoute dans 100 parties de sel surbasé 10,2 parties d'huile 200 Neutral. Après distillation à 145°C sous $0,665 \cdot 10^3$ Pa, on recueille d'une part, 38 parties de white spirit, et d'autre part 72,3 parties de sel surbasé de calcium sous la forme d'un solide couleur ambrée. Ses caractéristiques sont les suivantes:
– masse volumique: 1,046 g/cm³
– viscosité dynamique à 100°C: 1,25 Pascal.seconde
– TBN: 376
– teneur en calcium (en % en poids): 13,04 à 13,08

EXEMPLE 4

Conformément au mode opératoire décrit dans l'exemple 1, on fait réagir la charge suivante (en parties en poids ):
– acide constitué d'un mélange d'acides OXO en C8, C9 et C10 contenant au plus 10% en poids d'acide linéaire, au plus 10% en poids d'acides ramifiés sur le carbone $C_2$ et au moins 80% en poids d'acides ramifiés sur le carbone $C_3$ et/ou de rang supérieur et dont l'indice d'acide est égal à 368: 250
– hydroxyde de calcium: 128
avec un débit de dioxyde de carbone tel que le rapport massique horaire de dioxyde de carbone sur l'hydroxyde de calcium est égal à 1,1 et en présence de (en parties en poids):
– triéthylène glycol: 35
– octanoate de zinc: 1,2
– white spirit: 287
Au cours de la réaction, on récupère 29,6 parties d'eau. En fin de réaction, on recueille 671,6 parties de sel surbasé de calcium qui se présente sous la forme d'un liquide jaune clair limpide. Dans la seconde étape, on ajoute dans 100 parties de ce sel surbasé de calcium 7,1 parties d'huile paraffinique de marque commerciale 200 Neutral. Après distillation à 145°C sous $0,665 \cdot 10^3$ Pa, on recueille, d'une part, 43,1 parties de white spirit, et d'autre part, 64 parties de savons surbasés de calcium sous la forme d'un solide couleur ambrée. Ses caractéristiques sont les suivantes:
– masse volumique: 1,1 g/cm³
– viscosité dynamique à 100°C: 0,58 Pascal.seconde
– TBN: 399
– teneur en calcium (en % en poids): 14,07

EXEMPLE 5 (comparatif)

Conformément au mode opératoire défini dans l'exemple 2, on fait réagir la charge suivante (en parties en poids):
– acide linéaire heptanoïque: 260
– hydroxyde de calcium: 128
avec un débit de dioxyde de carbone tel que le rapport massique horaire de dioxyde de carbone sur l'hydroxyde de calcium est égal à 1,1 et en présence de (en parties en poids):
– triéthylène glycol: 35
– octanoate de zinc: 1,5
– white spirit: 293

Au cours de la réaction, cn récupère 36 parties d'eau. En fin de réaction, on obtient 681,5 parties d'un produit très visqueux blanchâtre qui s'avère infiltrable.

EXEMPLE 6 (comparatif)

Conformément au mode opératoire défini dans l'exemple 1, on fait réagir la charge suivante (en parties en poids):
– un mélange d'acides en C7 principalement ramifiés sur le carbone 2 et comprenant de l'éthyle-2 pentanoïque (60 à 70% en poids) et du méthyle-2-hexanoïque (15 à 25% en poids): 325
– hydroxyde de calcium: 160
avec un débit de dioxyde de carbone tel que le rapport massique horaire de dioxyde de carbone sur l'hydroxyde de calcium est égal à 1,1 et en présence de (en parties en poids):
– triéthylène glycol: 41,2
– octanoate de zinc: 1,5
– white spirit: 364,2

Au cours de la réaction, on récupère 45 parties d'eau. En fin de réaction, on obtient 846,9 parties d'un produit très visqueux et infiltrable.

EXEMPLE 7 (comparatif)

Conformément au mode opératoire défini dans l'exemple 1, on fait réagir la charge suivante (en parties en poids):
– acide 2-éthyl-hexanoïque: 360
– hydroxyde de calcium: 160
avec un débit de dioxyde de carbone tel que le rapport massique horaire de dioxyde de carbone sur l'hydroxyde de calcium est égal à 1,1 et en présence de (en parties en poids):
– triéthylène glycol: 41,2
– octanoate de zinc: 1,5
– white spirit: 329

Au cours de la réaction, on récupère 45 parties d'eau. En fin de réaction, on obtient 846,7 parties d'une masse réactionnelle visqueuse infiltrable.

EXEMPLE 8 (comparatif)

Conformément au mode opératoire défini dans l'exemple 1, on fait réagir la charge suivante (en parties en poids):
– acide diméthyl-2,2-hexanoïque: 344
– hydroxyde de calcium: 128
avec un débit de dioxyde de carbone tel que le rapport massique horaire de dioxyde de carbone sur l'hydroxyde de calcium est égal à 1,1 et en présence de (en parties en poids):
– triéthylène glycol: 35
– octanoate de zinc: 1,5
– white spirit: 220

Au cours de la réaction, on récupère 36 parties d'eau. En fin de réaction, on obtient 692,2 parties d'une masse réactionnelle visqueuse infiltrable.

EXEMPLE 9 (comparatif)

Conformément au mode opératoire décrit dans l'exemple 1, on fait réagir la charge suivante (en parties en poids):
– acide commercialisé sous la marque CEKANOIC et constitué d'un mélange d'isomères de l'acide octanoïque comprenant au plus 10% en poids d'acide linéaire, au plus 10% en poids d'acides ramifiés sur le carbone 2 et au moins 80% en poids d'acides ramifiés sur les carbones 3 et/ou de rang supérieur: 144
– hydroxyde de calcium: 95,4
– dioxyde de carbone avec un débit tel que le rapport massique horaire de dioxyde de carbone sur l'hydroxyde de calcium est égal à 1,1 et en présence de (en parties en poids):
– méthanol: 100
– white spirit: 261       ·
– octanoate de zinc: 1,2

Au cours de la réaction, on récupère 101,6 parties d'un mélange eau et méthanol. En fin de réaction on recueille 500 parties de sel surbasé de calcium qui se présente sous la forme d'un liquide jaune clair limpide. Dans la seconde étape, on ajoute dans 100 parties de ce sel surbasé de calcium, 5,1 parties d'huile paraffinique 200 Neutral. Après distillation à 145°C sous 0,665·10³ Pa, on recueille d'une part, 52,2 parties de white spirit et d'autre part, 51,3 parties de savon surbasé de calcium sous la forme d'un solide de couleur ambrée. Ses caractéristiques sont les suivantes:
— masse volumique: 1,12 g/cm³
— viscosité dynamique à 100°C: 1,3 Pascal.seconde
— Total Basic Number (TBN): 502
— teneur en calcium (en % en poids): 17,7

EXEMPLE 10 (comparatif)

Conformément au mode opératoire décrit dans l'exemple 1, on fait réagir la charge suivante (en parties en poids):
— acide n-octanoïque: 236
— hydroxyde de calcium: 128
— dioxyde de carbone avec un débit tel que le rapport massique horaire de dioxyde de carbone sur l'hydroxyde de calcium est égal à 1,1 et en présence de (en parties en poids):
— triéthylène glycol: 35
— white spirit: 286,3
— octanoate de zinc: 1,2
Au cours de la réaction, on ne récupère que 13 parties d eau. En fin de réaction, on obtient 673,5 parties d'une masse visqueuse infiltrable.

EXEMPLE 11 (comparatif) (avec une composition en acide de 75% d'acide iso-octanoïque et 25% d'acide ramifié sur le carbone en $C_2$)

Conformément au mode opératoire défini dans l'exemple 1, on fait réagir la charge suivante (en parties en poids):
— acide iso-octanoïque: 108
— acide 2-éthyl-hexanoïque: 36
— hydroxyde de calcium: 95,4
avec un débit de dioxyde de carbone tel que le rapport massique horaire de dioxyde de carbone sur l'hydroxyde de calcium est égal à 1,1 et en présence de (en parties en poids):
— méthanol: 59
— white spirit: 277,6
— octanoate de zinc: 1,2
Au cours de la réaction, on récupère 56 parties d'eau et de méthanol. En fin de réaction, on obtient 397,5 parties d'un produit jaunâtre, trouble. Après filtration le poids de résidu sur le filtre est égal à environ 25% du poids de sel de calcium surbasé.

EXEMPLE 12 (comparatif) (avec une composition en acide de 50% en linéaire et de 50% d'acide iso-octanoïque)

Conformément au mode opératoire défini dans l'exemple 1, on fait réagir la charge suivante (en parties en poids):
— acide n-octanoïque: 72
— acide iso-octanoïque: 72
— hydroxyde de calcium: 95,4
avec un débit de dioxyde de carbone tel que le rapport massique horaire de dioxyde de carbone sur l'hydroxyde de calcium est égal à 1,1 et en présence de (en parties en poids):
— méthanol: 50
— white spirit: 277,6
— octanoate de zinc: 1,2
Au cours de la réaction, on récupère 63,7 parties d'eau et de méthanol. En fin de réaction, on obtient 474,2 parties d'un produit liquide jaunâtre trouble après filtration, le poids de résidu sur le filtre est égal à environ 6% en poids de sel de calcium surbasé.

EXEMPLE 13 (avec une composition en acide de 40% en linéaire et 20% de ramifié en $C_2$ et 40% de ramifié en $C_3$

Conformément au mode opératoire décrit dans l'exemple 1, on fait réagir la charge suivante (en parties en poids):
— acide n-octanoïque: 57,6 (40% en poids du mélange d'acide)
— acide 2-éthyl-hexanoïque: 28,8 (20% en poids du mélange d'acide)
— acide 3,5,5-triméthylhexanoïque: 57,6 (40% en poids d'un mélange d'acide)

– hydroxyde de calcium: 95,4
– dioxyde de carbone avec un débit tel que le rapport massique horaire de dioxyde de carbone sur l'hydroxyde de calcium est égal à 1,1 et en présence de (en parties en poids):
– méthanol: 50
– white spirit: 277,6
– octanoate de zinc: 1,2
Au cours de la réaction, on récupère 67,3 parties d'un mélange eau et méthanol. En fin de réaction on recueille 500,9 parties de sel surbasé de calcium qui se présente sous la forme d'un liquide jaune clair limpide. Dans la second étape on ajoute à 100 parties de ce sel surbasé de calcium, 65,5 parties d'huile paraffinique 100 Neutral. Après distillation à 145°C sous 5 mm de mercure, on recueille d'une part, 55,4 parties de white spirit et d'autre part 100 parties de savon surbasé de calcium sous la forme d'une pâte translucide de couleur ambre et véhiculable à température ambiante.
Les caractéristiques sont les suivantes:
– masse volumique: 1,01 g/cm$^3$
– viscosité dynamique
. à 100°C: 0,280 Pascal.seconde
. à 20°C: 45 Pascal.seconde
– total Basic Number (TBN): 240
– teneur en calcium: 8,5%

EXEMPLE 14 (avec une composition en acide de 40% en linéaire et de 60% d'acide isooctanoïque)

En suivant le mode opératoire décrit dans l'exemple 1, on fait réagir la charge suivante (en parties en poids):
– acide n-octanoïque: 57,6
– acide iso-octanoïque: 86,4
– hydroxyde de calcium: 95,4
– dioxyde de carbone avec un débit tel que le rapport massique horaire de dioxyde de carbone sur l'hydroxyde de calcium est égal à 1,1 et en présence de (en parties en poids):
– méthanol: 50
– white spirit: 277,6
– octanoate de zinc: 1,2
Au cours de la réaction, on récupère 68 parties d'un mélange eau et méthanol. En fin de réaction, on recueille 500,2 parties de sel surbasé de calcium qui se présente sous la forme d'un liquide jaune clair limpide. Dans la seconde étape, on ajoute à 100 parties de ce sel surbasé de calcium, 13 parties d'huile paraffinique 200 Neutral.
Après distillation à 145°C sous 5 mm de mercure, on recueille d'une part, 54,3 parties de white spirit et d'autre part 58,7 parties de savon surbasé de calcium sous la forme d'un solide couleur ambrée.
Les caractéristiques sont les suivantes:
– masse volumique: 1,08 g/cm$^3$
– viscosité dynamique à 100°C: 1,14 Pascal. seconde
– total Basic Number (TBN): 425
– teneur en calcium: 15,1%

EXEMPLE 15 (avec une composition de 90% d'acide iso-octanoïque et 10% d'acide ramifié sur le carbone en C$_2$)

Conformément au mode opératoire décrit dans l'exemple 1, on fait réagir la charge suivante (en parties en poids):
– acide iso-octanoïque: 129,6
– acide 2-éthyl-hexanoïque: 14,4
– hydroxyde de calcium: 95,4
– dioxyde de carbone avec un débit tel que le rapport massique horaire de dioxyde de carbone sur l'hydroxyde de calcium est égal à 1,1 et en présence de (en parties en poids):
– méthanol: 50
– white spirit: 277,6
– octanoate de zinc: 1,2
Au cours de la réaction, on récupère 68 parties d'un mélange eau et méthanol. En fin de réaction, on recueille 500,2 parties de sel surbasé de calcium qui se présente sous la forme d'un liquide jaune clair limpide. Dans la seconde étape, on ajoute à 100 parties de ce sel surbasé de calcium, 16,5 parties d'huile paraffinique 100 Neutral.
Après distillation à 145°C sous 5 mm de mercure, on recueille d'une part, 55,4 parties de white spirit et d'autre part, 61,1 parties de savon surbasé sous la forme d'un solide translucide de couleur ambre.
Ses caractéristiques sont les suivantes:
– masse volumique: 1,085 g/cm$^3$

– viscosité dynamique à 100°C: 1,35 Pascal.seconde
– Total Basic Number (TBN): 438
– teneur en calcium: 15,6%

**Revendications**

1. Savons de calcium possédant une réserve de basicité élevée caractérisés en ce qu'ils comprennent du calcium dissous dans au moins une huile sous la forme de carbonate et de sels d'acides, lesdits acides étant constitués d'un mélange ou non d'acides organiques carboxyliques saturés de 7 à 13 atomes de carbone possédant les caractéristiques suivantes:
– une teneur en acides linéaires comprise entre 0 et 40% en poids,
– une teneur en acides ramifiés sur le carbone 2 comprise entre 0 et 20% en poids,
– une teneur en acides, mono ou polysubstitués sur le carbone 3 et/ou les carbones de rang supérieur, comprise entre 40 et 100% en poids.

2. Savons de calcium selon la revendication 1, caractérisés en ce que lesdits acides organiques sont des acides organiques carboxyliques saturés en C8, C9 ou C10 constitués d'un mélange d'isomères dont la teneur en acides linéaires n'excède pas 10% en poids, dont la teneur en acides ramifiés sur le carbone 2 n'excède pas 10% en poids, la teneur en acides ramifiés sur le carbone 3 et/ou les carbones de rang supérieur est d'au moins 80% en poids.

3. Savons de calcium selon la revendication 1, caractérisés en ce que l'acide carboxylique est un mélange d'isomères de l'acide octanoïque comprenant au plus 10% en poids d'acide n-octanoïque, au plus 10% en poids d'acides ramifiés sur le carbone 2 et au moins 80% en poids d'acides ramifiés sur le carbone 3 et/ou les carbones de rang supérieur.

4. Savons de calcium selon la revendication 1, 2 ou 3 caractérisés en ce que l'huile est constituée d'au moins une huile naturelle et/ou au moins d'une huile semi-synthétique et/ou d'au moins une huile de synthèse.

5. Procédé de préparation de savons surbasés de calcium tels que définis dans la revendication 1, selon lequel on fait réagir sous agitation un oxyde et/ou un hydroxyde de calcium avec du dioxyde de carbone que l'on fait barboter dans le milieu réactionnel et au moins un acide organique carboxylique, en présence d'au moins un promoteur favorisant la fixation du dioxyde de carbone et d'au moins un catalyseur, en ce que l'on élimine l'eau formée, procédé caractérisé en ce que la réaction est effectuée dans au moins un solvant organique à une température comprise entre 80 et 120°C et en ce que ledit acide est un acide organique carboxylique saturé, de 7 à 13 atomes de carbone, dont la teneur en acides linéaires est inférieure ou égale à 40% en poids, dont la teneur en acides ramifiés sur le carbone 2 est inférieure ou égale à 20% en poids et dont la teneur en acides ramifiés sur le carbone 3 et/ou les carbones de rang supérieur est égale ou supérieure à 40% en poids et en ce que, en fin de réaction, on substitue au solvant organique une huile ou un mélange d'huiles choisi parmi les huiles naturelles et/ou les huiles semi-synthétiques et/ou les huiles de synthèse.

6. Procédé selon la revendication 5, caractérisé en ce que, pendant la réaction, on évapore le solvant organique et en ce que on le recycle dans le milieu réactionnel de manière à y occasionner un barbotage favorable à la réaction.

7. Procédé selon la revendication 5 ou 6, caractérisé en ce que le solvant organique qui est utilisé lors de la réaction est un solvant non polaire choisi parmi le naphta, l'hexane, le kérosène, le benzène, le toluène, le xylène ou un mélange d'hydrocarbures paraffiniques d'origine minérale ou de synthèse comportant de préférence une faible proportion d'hydrocarbures aromatiques et/ou naphteniques tel que le white spirit ou un solvant organique polaire choisi parmi les alcools, par exemple le butanol-1, le butanol-2, l'éthylène-glycol, le propylène-glycol, l'éther monométhylique de l'éthylène glycol, l'éther diméthylique de l'éthylène glycol, le diéthylène glycol et ses éthers, des mélanges d'alcools dérivés de la paraffine ou la méthyléthylcétone.

8. Procédé selon l'une quelconque des revendications 5 à 7, caractérisé en ce que le promoteur pour la fixation du dioxide de carbone est le méthanol.

**Patentansprüche**

1. Kalziumseifen mit hochbasischer Reserve, dadurch gekennzeichnet, daß sie in mindestens einem Öl gelöstes Kalzium in Form von Karbonat und Salzen von Säuren enthalten, wobei die genannten Säuren von in Mischung oder nicht in Mischung vorliegenden gesättigstoffatomen gebildet sind, welche folgende Eigenschaften besitzen:
– einen Gehalt an linearen Säuren zwischen 0 und 40 Gew.-%,
– einen Gehalt an am Kohlenstoff 2 verzweigten Säuren zwischen 0 und 20 Gew.-%, und
– einen Gehalt an Säuren, mono- oder polysubstituiert am Kohlenstoff 3 und/oder an den Kohlenstoffen höherer Zahl, zwischen 40 und 100 Gew.-%.

2. Kalziumseifen nach Anspruch 1, dadurch gekennzeichnet, daß die genannten organischen Säuren an C8, C9 oder C10 gesättigte organische Karbonsäuren sind und von einer Mischung an Isomeren gebildet sind, deren Gehalt an linearen Säuren 10 Gew.-% nicht übersteigt, wobei der Gehalt an am Kohlenstoff 3 und/oder an den Kohlenstoffen höherer Zahl verzweigte Säuren mindestens 80 Gew.-% beträgt.

3. Kalziumseifen nach Anspruch 1, dadurch gekennzeichnet, daß die Karbonsäure eine Mischung aus Isomeren der Oktansäure ist, welche höchstens 10 Gew.-% am n-Oktansäure, höchstens 10 Gew.-% am Kohlenstoff 2 verzweigte Säuren und mindestens 80 Gew.-% am Kohlenstoff 3 und/oder an den Kohlenstoffen höherer Zahl verzweigte Säuren enthält.

4. Kalziumseifen nach Anspruch 1, 2 oder 3, dadurch gekennzeichnet, daß das Öl von mindestens einem natürlichen Öl und/oder mindestens einem halbsynthetischen Öl und/oder mindestens einem synthetischen Öl gebildet ist.

5. Verfahren zur Herstellung von überbasischen Kalziumseifen, wie sie in Anspruch 1 definiert sind, bei welchem man unter Rühren ein Oxid und/oder ein Hydroxid des Kalziums mit Kohlendioxid, das man in das Reaktionsmilieu einbläst, und mindestens einer organischen Karbonsäure in Gegenwart mindestens eines die Fixierung des Kohlendioxids begünstigenden Promotors und mindestens eines Katalysators reagieren läßt, und das gebildete Wasser entfernt, dadurch gekennzeichnet, daß die Reaktion in mindestens einem organischen Lösungsmittel bei einer Temperatur zwischen 80° und 120°C durchgeführt wird, daß die genannte Säure eine gesättigte organische Karbonsäure mit 7 bis 13 Kohlenstoffatomen ist, deren Gehalt an linearen Säuren weniger als oder gleich 40 Gew.-% beträgt, deren Gehalt an am Kohlenstoff 2 verzeigten Säuren weniger als oder gleich 20 Gew.-% beträgt und deren Gehalt an dem Kohlenstoff 3 und/oder an den Kohlenstoffen höherer Zahl verzweigten Säuren mehr als oder gleich 40 Gew.-% beträgt, und daß man am Ende der Reaktion das organische Lösungsmittel durch ein Öl oder eine Mischung an Ölen ersetzt, welche Öle ausgewählt sind aus der Gruppe bestehend aus den natürlichen Ölen und/oder den halbsynthetischen Ölen und/oder den synthetischen Ölen.

6. Verfahren nach Anspruch 5, dadurch gekennzeichnet, daß man während der Reaktion das organische Lösungsmittel verdampft und daß man es derart in das Reaktionsmilieu rückführt, daß es dort ein die Reaktion begünstigendes Durchblasen verursacht.

7. Verfahren nach Anspruch 5 oder 6, dadurch gekennzeichnet, daß das während der Reaktion verwendete organische Lösungsmittel ein nicht polares Lösungsmittel, das ausgewählt ist aus der Gruppe bestehend aus Naphtha, Hexan, Kerosin, Banzol, Toluol, Xylol, oder eine Mischung aus Paraffin-Kohlenwasserstoffen mineralischen oder synthetischen Ursprungs ist, welche vorzugsweise einen geringen Anteil an aromatischen und/oder naphthenischen Kohlenwasserstoffen enthält, wie Leichtbenzin oder ein polares organisches Lösungsmittel, das ausgewählt ist aus der Gruppe bestehend aus den Alkoholen, z.B. Butanol-1, Butanol-2, Äthylenglykol, Propylenglykol, Äthylenglykolmonomethyläther, Äthylenglykoldimethyläther, Diäthylenglykol und seinen Äthern, Mischungen von von Paraffin abstammenden Alkoholen und Methyläthylketon.

8. Verfahren nach irgendeinem der Ansprüche 5 bis 7, dadurch gekennnzeichnet, daß der Promotor zur Fixierung des Kohlendioxids Methanol ist.

**Claims**

1. Calcium soaps having a high basicity reserve, characterized in that they contain calcium dissolved in at least one oil in the form of carbonate and of salts of acids, the said acids consisting of a mixture or otherwise of saturated organic carboxylic acids containing from 7 to 13 carbon atoms, having the following characteristics:
a linear acid content of between 0 and 40% by weight,
a content of acids branched on carbon 2 of between 0 and 20% by weight, and
a content of acids which are mono- or polysubstituted on carbon 3 and/or on carbons of higher rank, of between 40 and 100% by weight.

2. Calcium soaps according to Claim 1, characterized in that the said organic acids are saturated C8, C9 or C10 organic carboxylic acids consisting of an isomeric mixture in which the content of linear acids does not exceed 10% by weight, in which the content of acids branched on carbon 2 does not exceed 10% by weight, and the content of acids branched on carbon 3 and/or the carbons of higher rank is at least 80% by weight.

3. Calcium soaps according to Claim 1, characterized in that the carboxylic acid is a mixture of isomers of octanoic acid containing at most 10% by weight of n-octanoic acid, at most by 10% weight of acids branched on carbon 2 and at least 80% by weight of acids branched on carbon 3 and/or the carbons of higher rank.

4. Calcium soaps according to Claim 1, 2 or 3 characterized in that the oil consists of at least one natural oil and/or at least one semisynthetic oil and/or at least one synthetic oil.

5. Process for the preparation of calcium superbase soaps such as defined in Claim 1, according to which a calcium oxide and/or hydroxide is reacted, with stirring, with carbon dioxide which is bubbled through the reaction medium and at least one organic carboxylic acid, in the presence of at least one promoter which promotes carbon dioxide fixation and of at least one catalyst, process characterized in that the water formed is removed, in that the reaction is performed in at least one organic solvent at a temperature of between 80 and 120°C and in that the said acid is a saturated organic carboxylic acid containing 7 to 13 carbon atoms, in which the content of linear acids is less than or equal to 40% by weight, in which the content of acids branched on carbon 2 is less than or equal to 20% by weight and in which the content of acids branched on carbon 3 and/or the carbons of higher rank is equal to or higher than 40% by weight and in that, when the reaction has ended, the organic solvent is replaced by an oil or a mixture of oils chosen from natural oils and/or semisynthetic oils and/or synthetic oils.

6. Process according to Claim 5, characterized in that, during the reaction, the organic solvent is evaporated off and in that it is recycled into the reaction mixture so as to produce therein a bubbling action which promotes the reaction.

7. Process according to Claim 5 or 6, characterized in that the organic solvent which is used during the reaction is a nonpolar solvent chosen from naphtha, hexane, kerosene, benzene, toluene, xylene or a mixture of paraffinic hydrocarbons of mineral or synthetic origin, preferably comprising a low proportion of aromatic and/or naphthenic hydrocarbons, such as white spirit, or a polar organic solvent chosen from alcohols, for example 1-butanol, 2-butanol, ethylene glycol, propylene glycol, ethylene glycon monomethyl ether, ethylene glycol dimethyl ether, diethylene glycol and its ethers, mixtures of alcohols, derived from paraffin or methyl ethyl ketone.

8. Process according to any one of Claims 5 to 7, characterized in that the promoter for the fixation of carbon dioxide is methanol.